# EUROPÄISCHE PATENTANMELDUNG

(11) **EP 1 484 627 A1**
(43) Veröffentlichungstag der Anmeldung: **08.12.2004**
(21) Anmeldenummer: 04013269.8
(22) Anmeldetag: 04.06.2004
(51) Int. Cl.: G02B 21/18, G02B 21/22

(54) **Mikroskop**

(30) Priorität: 05.06.2003 DE 10325575
(71) Anmelder: Leica Microsystems Schweiz AG, 9435 Heerbrugg (CH)
(72) Erfinder: Sander, Ulrich, 9445 Rebstein (CH)
(74) Vertreter: Hössle Kudlek & Partner

(57) **Zusammenfassung**

Mikroskop, insbesondere Stereomikroskop, zur Simultanbeobachtung eines Objektes durch einen ersten und einen zweiten Beobachter, mit einem eine optische Achse (2a) aufweisenden Hauptobjektiv (2) und Umlenkelementen (5, 6, 7) zum Umlenken eines parallel zu der optischen Achse (2a) des Hauptobjektivs (2) verlaufenden Beobachtungsstrahlenganges in eine erste Ebene I, welche sich unter einem Winkel, insbesondere im wesentlichen senkrecht, zu der optischen Achse (2a) des Objektivs (2) erstreckt und anschließend in eine zweite Ebene II, welche sich im wesentlichen parallel zu der ersten Ebene I oberhalb von dieser erstreckt, und einer Auskopplungseinrichtung (7) zur Auskopplung eines Beobachtungsstrahlenganges des zweiten Beobachters von dem Beobachtungsstrahlengang des ersten Beobachters, wobei eine Umlenkeinrichtung (20) zur Umlenkung des Beobachtungsstrahlenganges des zweiten Beobachters in eine dritte Ebene III, welche sich im wesentlichen parallel zu der ersten und der zweiten Ebene I, II erstreckt, und die zweite Ebene II zwischen der ersten Ebene I und der dritten Ebene III liegt.

## Beschreibung

Die vorliegende Erfindung betrifft ein Mikroskop, insbesondere ein Stereomikroskop, nach dem Oberbegriff des Patentanspruchs 1.

Neue Operationstechniken in der neuen Neurochirurgie erfordern die Beobachtung eines zu operierenden Bereiches gleichzeitig durch einen Hauptoperateur und einen Assistenten. Hierbei sollten Hauptoperateur und Assistent das gleiche Blickfeld beobachten können. Diese Anforderung ist in der Praxis dadurch erschwert, daß das Mikroskop in allen Raumrichtungen orientiert sein kann bzw. in allen Raumrichtungen orientiert bedienbar sein muß.

Der japanische Hersteller Olympus hat ein in der Neurochirurgie einsetzbares Mikroskop entwickelt, wobei dort die Lösung im Wesentlichen auf dem Einsatz eines Vergrößerungssystems beruht, welches keine stereoskopische Aufspaltung verursacht. Dies erleichtert zwar einerseits die Entwicklung eines solchen Mikroskops, verursacht aber schwerwiegende Mängel, die in bestimmten Anwendungsbereichen durch die Benutzer kaum akzeptiert werden. Ein dort auftretender Mangel besteht beispielsweise in einem mit der Vergrößerung variierenden räumlichen Eindruck.

Aus der US 2001/0010592 A1 ist ein Stereomikroskop bekannt, bei welchem eine Aufspaltung der Beobachtungsstrahlengänge für Hauptoperateur und Assistenten bereits vor jeweiligen Objektiv- und Vergrößerungssystemen ausgeführt wird. Dort werden die Beobachtungsstrahlengänge für den Hauptoperateur nach Durchgang durch ein erstes Hauptobjektiv in die Waagerechte umgelenkt, wobei ein entsprechendes Vergrößerungssystem für den Hauptoperateur waagerecht angeordnet ist. Das Assistenten-Mikroskop wird unterhalb des Hauptmikroskops angeordnet. Als nachteilig hierbei erweist sich, daß der freie Arbeitsabstand unterhalb des Mikroskops (d. h. der Abstand zwischen dem Mikroskop und dem zu beobachtenden Objekt) reduziert und die Bauhöhe des Mikroskops insgesamt vergrößert wird. Da auch für das Assistenten-Mikroskop ein separates Hauptobjektiv benötigt wird, erweist sich dieses Mikroskop ferner als relativ aufwendig in der Bereitstellung der jeweiligen optischen Komponenten.

Aufgabe der Erfindung ist die Bereitstellung eines Mikroskops, insbesondere eines Stereomikroskops, zur Simultanbeobachtung für einen ersten und einen zweiten Beobachter (Hauptoperateur und Assistent), bei dem der freie Arbeitsabstand unterhalb des Mikroskops möglichst wenig eingeschränkt wird, und dessen Bauhöhe möglichst klein ist.

Diese Aufgabe wird gelöst durch ein Mikroskop mit den Merkmalen des Patentanspruchs 1.

Erfindungsgemäß ist nun ein beispielsweise in der Neurochirurgie einsetzbares Mikroskop, insbesondere Stereomikroskop, zur Verfügung gestellt, bei dem der freie Arbeitsabstand unterhalb des Mikroskops maximal ist, und in keiner Weise durch die Positionierung eines Assistenten-Mikroskops eingeschränkt wird. Das erfindungsgemäß zur Verfügung gestellte Mikroskop weist gegenüber herkömmlichen Mikroskopen auch eine verringerte Bauhöhe auf.

Mit dem erfindungsgemäßen Mikroskop ist ferner gewährleistet, das sowohl ein Hauptoperateur als auch ein Assistent einen stereoskopischen Bildeindruck unabhängig von einer eingestellten Vergrößerung erhalten, wobei das Bildfeld für den Hauptoperateur und den Assistenten im wesentlichen gleich ist. Ferner erweist sich als vorteilhaft, daß gemäß dem erfindungsgemäßen Mikroskop lediglich ein Hauptobjektiv verwendet werden muß, da die Aufspaltung der Strahlengänge in Hauptoperateur-Strahlengänge und Assistenten-Strahlengänge erst nach dem Durchgang der Strahlengänge durch das Hauptobjektiv durchgeführt wird.

Die dargestellten Ziele werden im wesentlichen dadurch erreicht, daß die Strahlengänge nach ihrem Durchgang durch das Hauptobjektiv Umlenkungen erfahren, so daß sich insgesamt drei im wesentlichen parallele Strahlengang-Ebenen ergeben. Hierbei erfolgt die Auskopplung des Strahlengangs für den Assistenten bzw. das Assistentenmikroskop derart, daß dieser in der obersten der drei Ebenen verläuft, d.h. in der Ebene mit maximalem Abstand zum zu beobachtenden Objekt.

Vorteilhafte Ausgestaltungen des erfindungsgemäßen Mikroskops sind Gegenstand der Unteransprüche.

Gemäß einer besonders bevorzugten Ausführungsform des erfindungsgemäßen Mikroskops ist ein Vergrößerungssystem für den ersten Beobachter (Hauptoperateur) in der zweiten bzw. mittleren Mikroskopebene, und ein Vergrößerungssystem für den zweiten Beobachter (Assistent) in der dritten (obersten) Mikroskopebene angeordnet. Durch diese Maßnahme, die Vergrößerungssysteme für den ersten und den zweiten Beobachter in den insbesondere im wesentlichen horizontal verlaufenden zweiten bzw. dritten Ebenen anzuordnen, kann die Bauhöhe des Mikroskops insgesamt sehr klein gehalten werden. Dadurch, daß für den ersten und den zweiten Beobachter jeweils unabhängige Vergrößerungssysteme vorgesehen sind, können für den ersten Beobachter (Hauptoperateur) und den zweiten Beobachter (Assistent) bei Bedarf unterschiedliche Vergrößerungen eingestellt werden. Es sei angemerkt, dass die horizontale Erstreckung der Mikroskopebenen, insbesondere bei Einsatz des erfindungsgemäßen Mikroskops auf dem Gebiet der Neurochirurgie, lediglich eine bevorzugte Orientierung darstellt. Prinzipiell soll das erfindungsgemäße Mikroskop auch derart orientierbar sein, dass sich die Ebenen, bevorzugt parallel zueinander, geneigt im Raum erstrecken.

Zweckmäßigerweise weist wenigstens eines der Vergrößerungssysteme für den ersten oder zweiten Beobachter eine Zoom-Optik auf. Mit einer derartigen Zoom-Optik ist eine gewünschte Vergrößerung über einen vorgegebenen Zoom-Bereich individuell in gewünschter Weise einstellbar.

Gemäß einer weiteren bevorzugten Ausführungsform des erfindungsgemäßen Mikroskops sind die Beobachtungsstrahlengänge in der zweiten Ebene und/oder der dritten Ebene verschwenkbar. Durch diese Maßnahme ist beispielweise das Assistentenmikroskop frei in allen Raumrichtungen einsetzbar, wodurch eine Behinderung des Hauptoperateurs wirksam vermieden werden kann.

Zweckmäßigerweise ist wenigstens eine der Umlenkeinrichtungen, mittels der die Beobachtungsstrahlengänge von einer der Ebenen in eine andere Ebene umlenkbar sind, halbdurchlässig ausgebildet. Durch diese Maßnahme ist eine Auskopplung der Assistenten-Strahlengänge ohne Einengung von Strahlengangpupillen möglich (physikalische Strahlenteilung). Hierdurch können Vignettierungen wirksam vermieden werden.

Gemäß einer weiteren bevorzugten Ausführungsform des erfindungsgemäßen Mikroskops sind die Vergrößerungssysteme für den ersten und zweiten Beobachter mechanisch und/oder elektrisch miteinander gekoppelt. Mit dieser Maßnahme kann sichergestellt werden, dass insbesondere bei Verwendung von Zoom-Systemen als Vergrößerungssystem gleiche Vergrößerungen für beide Beobachter einstellbar sind, bzw. eingestellte Vergrößerungen synchron zueinander veränderbar sind. Diese Maßnahme kann beispielsweise durch gekoppelte Antriebseinheiten für die beiden Zoom-Systeme realisiert sein.

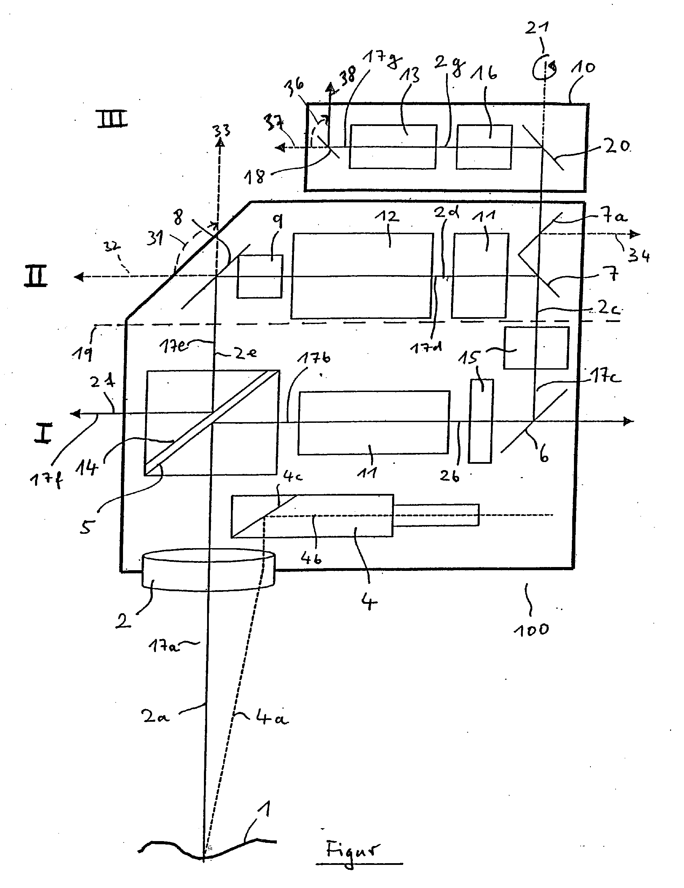

Die Erfindung wird nun anhand der beigefügten Zeichnung weiter beschrieben. In dieser zeigt:
die einzige Figur eine bevorzugte Ausführungsform des erfindungsgemäßen Mikroskops in schematischer seitlicher Schnittansicht.

Eine bevorzugte Ausführungsform eines als Stereomikroskop ausgebildeten erfindungsgemäßen Mikroskops ist in der Figur insgesamt mit 100 bezeichnet. Mittels des Mikroskops 100 soll beispielsweise ein neurologisches Objekt 1 beobachtet werden. Eine Beleuchtungseinrichtung, welche das Objekt 1 entlang einer Beleuchtungsachse 4a beleuchtet, ist insgesamt mit 4 bezeichnet.

Das Objekt 1 wird über ein Hauptobjektiv 2 in das Mikroskop 100 abgebildet. Die optische Achse des Hauptobjektivs 2 ist mit 2a bezeichnet. Im dargestellten Ausführungsbeispiel verläuft sie im wesentlichen vertikal, wobei darauf hingewiesen sei, dass das Mikroskop 100, und somit auch die optische Achse 2a, in allen Raumrichtungen orientierbar sein kann. Das Hauptobjektiv wird von einem Beobachtungsstrahlengang 17a, welcher entlang der optischen Achse 2a verläuft, durchsetzt. Der Beobachtungsstrahlengang 17a wird anschließend über ein Umlenkelement 5 in eine erste Mikroskopebene I umgelenkt. Die optische Achse in der ersten Mikroskopebene I ist mit 2b, und der umgelenkte Strahlengang mit 17b bezeichnet. In der ersten Mikroskopebene I sind optische Komponenten 11, 15 angeordnet, welche beispielsweise Zwischenabbildungen des Beobachtungsstrahlengangs 17b verursachen. Der in der ersten Mikroskopebene I verlaufende Beobachtungsstrahlengang 17b wird anschließend über ein Umlenkelement 6 entlang einer Achse 2c in die Vertikale, und anschließend über ein weiteres Umlenkelement 7 in eine zweite, im wesentlichen horizontal und parallel zu der ersten Mikroskopebene I verlaufende zweite Mikroskopebene II umgelenkt. In den Strahlengang 17b sowie den mit 17c bezeichneten vertikalen Strahlengang zwischen den Umlenkelementen 6 und 7 können optische Bauelemente 15, wie beispielsweise Dateneinspiegelungen, Shutter, Filter, transparente Displays, Strahlumlenksysteme oder Bildaufrichter eingefügt sein. In den Strahlengang 17c können auch optische Komponenten, welche Zwischenabbildungen verursachen, eingebracht werden. Derartige Komponenten wurden bereits erwähnt und mit Bezugszeichen 11 bezeichnet. Derartige Komponenten sind für den vertikalen Strahlengang 17c nicht explizit dargestellt. Es sei der Vollständigkeit halber darauf hingewiesen, dass die Reihenfolge der Komponenten 11 und 15, wie sie in der Figur dargestellt ist, nur beispielhaft ist. Es wäre gleichfalls möglich, beispielsweise entlang des Beobachtungsstrahlengangs 17b zunächst die Komponenten 15, und anschließend die Komponenten 11 anzuordnen. Gleiches gilt selbstverständlich für die Anordnung dieser Komponenten entlang der Beobachtungsstrahlengänge 17c oder 17d. Der in der zweiten Mikroskop-Ebene verlaufende Beobachtungsstrahlengang , mit 17d bezeichnet, passiert zunächst weitere optische Komponenten, welche für Zwischenabbildungen sorgen und wiederum insgesamt mit 11 bezeichnet sind. Wie bereits erwähnt, können auch entlang des Beobachtungsstrahlenganges 17d optische Komponenten, wie sie oben mit Bezugszeichen 15 bezeichnet wurden, angeordnet sein. Diese Komponenten sind entlang einer optischen Achse 2d angeordnet. Anschließend an diese optischen Komponenten 11 trifft der Beobachtungsstrahlengang 17d auf ein erstes Vergrößerungssystem 12 für einen ersten Beobachter (Hauptoperateur). Dieses Vergrößerungssystem 12, welches zweckmäßigerweise als Zoom-System ausgebildet ist, weist zwei Beobachtungskanäle auf, und teilt somit den die optischen Bauteile 11 verlassenen Beobachtungsstrahlengang 17d in zwei stereoskopische Strahlengänge für den ersten Beobachter (Hauptoperateur).

Hinter dem Vergrößerungssystem 12 können weitere optische Komponenten eingefügt sein. Beispielweise ist in der dargestellten Ausführungsform ein optischer Teiler 9 zur Dokumentation vorgesehen.

Über ein weiteres Umlenkelement 8 sind die stereoskopischen Beobachtungsstrahlengänge wieder nach unten, beispielsweise in die erste Mikroskop-Ebene I ablenkbar. (Beobachtungsstrahlengänge 17e, optische Achse 2e). Hier ist ein weiteres Umlenkelement 14 angeordnet, welches die Beobachtungsrichtung bzw. Einblickrichtung für den ersten Beobachter (Hauptoperateur) entlang einer optischen Achse 2f definiert (Beobachtungsstrahlengänge 17f). Sich an das Umlenkelement 14 anschließende Binokulartuben für den Hauptbeobachter sind zur Vereinfachung der Darstellung nicht gezeigt. Um dem Hauptoperateur auch bei schräg im Raum positioniertem Mikroskop einen physiologisch günstigen Einblick (d. h. einen im wesentlichen horizontalen Einblick) zu gewährleisten ist zweckmäßigerweise das Umlenkelement 14 um eine senkrecht in die Zeichenebene hinein verlaufende Achse drehbar, wobei ferner der (nicht gezeichnete) Binokulartubus um die optische Achse 2f drehbar ist.

Alternativ zu einer Beobachtung in der ersten Ebene kann auch auf das Umlenkelement 8 verzichtet werden, wodurch eine entsprechende Beobachtung für den Hauptoperateur in der zweiten Ebene in einfacher Weise realisierbar ist.

Auf das Umlenkelement 8 kann, wie erwähnt, auch verzichtet werden, wobei in diesem Fall in einfacher Weise ein Einblick in der zweiten Mikroskopebene II für den Hauptoperateur möglich ist. Das Umlenkelement 8 kann ferner derart ausgebildet sein, dass ein wahlweiser Einblick in der zweiten Ebene II oder der ersten Ebene I für den ersten Beobachter möglich ist. In diesem Zusammenhang sei darauf hingewiesen, dass mittels einer geeigneten Ausbildung des Umlenkelements 8 ein variabler Einblickwinkel realisierbar ist. Der Winkelbereich, über den dieser Einblick verschwenkbar ist, ist mittels des gekrümmten Pfeiles 31 veranschaulicht, welcher sich zwischen den Pfeilen 32 und 33 erstreckt. Eine spezielle Ausführungsform des Umlenkelements 8, mit der eine derartige Verschwenkung des Einblicks möglich ist, ist beispielsweise in der in dem Prospekt der Firma Leica "Leica M 680 2x2", Drucklegung IV 2000, 10M16801de und in der US 6,172,804 B1 beschrieben. Der Einblick in der zweiten Ebene ist mittels des gestrichelt gekennzeichneten Pfeils 32, der eine Verlängerung der Achse 2d darstellt, veranschaulicht. Ein senkrechter Einblick ist mittels des Pfeiles 33 veranschaulicht.

Die erfindungsgemäß vorgesehene Strahlengang-Auskopplung für einen zweiten Beobachter (Assistenten) wird mit Hilfe des Umlenkelements 7 erreicht, welches halbdurchlässig ausgebildet ist. Somit wird ein Teil des auf das Umlenkelement 7 auftreffenden Beobachtungsstrahlenganges nicht abgelenkt, sondern verläuft weiter entlang der optischen Achse 2c und trifft auf ein weiteres Umlenkelement 20, welches in einem insgesamt mit 10 bezeichneten Assistentenmikroskop vorgesehen ist. Das Umlenkelement 20 bewirkt die Umlenkung des Beobachtungsstrahls in eine dritte Mikroskopebene III (Beobachtungsstrahlengang 17g), in der zusätzliche optische Bauelemente für den Assistenten, insgesamt mit 16 bezeichnet, und ein zweites Vergrößerungssystem für den Assistenten, mit 13 bezeichnet, vorgesehen sind. Das optische Element 16 kann beispielsweise als Bildaufrichter ausgebildet sein. Ferner sind hier optische Elemente, welche oben bereits mit Bezugszeichen 11 bzw. 15 versehen wurden, einbringbar. Die optische Achse des Vergrößerungssystems 13 ist mit 2g bezeichnet.

Das Vergrößerungssystem 13 für den Assistenten ist zweckmäßigerweise in ähnlicher Weise wie das Vergrößerungssystem 12 für den Hauptoperateur aufgebaut, d.h. hier erfolgt eine Aufteilung des Beobachtungsstrahleingangs 17g in zwei stereoskopische Beobachtungsstrahlengänge. Auch das Vergrößerungssystem 13 für den Assistenten ist zweckmäßigerweise als Zoom-System ausgestaltet. Es ist möglich, die Vergrößerungssysteme 12, 13 mit jeweils unterschiedlichen, oder aber auch gleichen Vergrößerungsbereichen auszubilden. Hierbei kann eine elektrische oder mechanische Kopplungseinrichtung vorgesehen sein, mittels der beispielsweise gleiche Vergrößerungen der beiden Vergrößerungssysteme gewährleistet werden können.

Anschließend an das Vergrößerungssystem 13 sind (nicht dargestellt) Binokulartuben für den Assistenteneinblick vorgesehen. Dieser Assistenteneinblick wird vorteilhafterweise mittels eines drehbaren Umlenkelements 18 variabel gestaltet, wobei zu diesem Zwecke das Umlenkelement 18 analog zu dem Umlenkelement 8 ausgebildet sein kann. Auch hier ist der Winkelbereich, über den der Assistenteneinblick verschiebbar ist, mittels eines gekrümmten Pfeils 36 dargestellt, welcher sich analog zu dem Pfeil 31, zwischen Pfeilen 37 und 38 erstreckt. Ferner ist der Binokulartubus für den Assistenten um die Achse 2g drehbar ausgebildet. Das Assistentenmikroskop 10 ist ferner um die Achse 2c drehbar ausgebildet. Dadurch ist ein beispielsweise um 360° drehbarer Einblick für den Assistenten in der dritten Mikroskopebene III zur Verfügung gestellt. Pfeil 37 stellt einen horizontalen Einblick bezüglich der dritten Ebene III des Mikroskops dar, Pfeil 36 einen vertikalen Einblick.

Zwischen den Umlenkelementen 7 und 20 kann ein weiteres, insbesondere halbdurchlässiges Umlenk- bzw. Strahlenteilerelement 7a vorgesehen sein, mit dem eine Strahlengangauskopplung, beispielsweise für Dokumentationszwecke, realisierbar ist (mittels gestrichelten Pfeils 34 veranschaulicht).

Es ist ebenfalls möglich, einen drehbaren Einblick für den Hauptoperateur zur Verfügung zu stellen. Hierzu muß lediglich der oberhalb der gestrichelten Linie 19 liegende Bereich des Mikroskops gegenüber dem unterhalb dieser Linie liegenden Bereich drehbar ausgebildet sein (wie durch Drehpfeil 21 angedeutet). Diese Drehbarkeit ist aufgrund der Tatsache, daß eine stereoskopische Aufspaltung der Beobachtungsstrahlengänge für den Hauptoperateur bzw. den Assistenten erst in den Vergrößerungssystemen 12 bzw. 13 durchgeführt wird, durch Drehung der Umlenkelemente 7 bzw. 20 um die optische Achse 2c in einfacher Weise realisierbar.

Erfindungsgemäß sind nun erstmals sämtliche Anforderungen an ein in der Neurochirurgie einsetzbares Mikroskop erfüllt. So schauen Assistent und Hauptoperateur durch ein gemeinsames Hauptobjektiv, wobei der Arbeitsabstand durch den Assistenten in keiner Weise eingeschränkt wird. Ferner wird die Bauhöhe des Mikroskops durch den Assistenten bzw. das Assistentenmikroskop nicht vergrößert. Zusatzbauelemente 9, 15, 11, 16 vergrößern ebenfalls nicht die Bauhöhe, sondern ermöglichen eine bessere Ausbalancierung des Mikroskops. Zusatzbauelemente 9, 15, 11, 16 können zusätzlich zu den in der Figur dargestellten Positionen an beliebig anderen, hier nicht im einzelnen spezifizierten Positionen entlang der Beobachtungsstrahlengänge 17a bis 17g vorgesehen sein. Beide Beobachter weisen ein separates Vergrößerungs- bzw. Zoom-System mit der Möglichkeit der klassischen stereoskopischen Aufspaltung (echter räumlicher Eindruck) auf. Ferner ist das Assistentenmikroskop frei in allen Raumrichtungen einsetzbar und behindert somit nicht den Hauptoperateur. Es sei schließlich darauf hingewiesen, dass auch die Anordnung der Beleuchtungseinrichtung 4, wie sie in der Figur dargestellt ist, lediglich beispielhaft ist. Es ist beispielsweise vorteilhaft möglich, die Beleuchtungseinrichtung 4 im Bereich der ersten Mikroskopebene I zwischen den Umlenkelementen 5 und 6 anzuordnen, wobei hier das aus dem Beleuchtungselement austretende Licht zweckmäßigerweise mittels des Umlenkelements 5 in geeigneter Weise auf das Hauptobjektiv 2 bzw. das zu beobachtende Objekt 1 umlenkbar sein kann. Das Umlenkelement 5 könnte hier bei entsprechender Anordnung gegebenenfalls das Umlenkelement 4c der Beleuchtungseinrichtung 4 ersetzen. Andererseits wäre es möglich, die Haupterstreckungsachse 4b der Beleuchtungseinrichtung 4 senkrecht auszubilden, und mittels eines entsprechend ausgerichteten Umlenkelements 4c der Beleuchtungseinrichtung 4 Licht auf das Umlenkelement 5 zu lenken. Mit dieser Maßnahme kann gegebenenfalls die Bauhöhe des Mikroskops weiter verkleinert werden.

### Bezugszeichenliste:

- 1: Objekt
- 2: Hauptobjektiv
- 2a: optische Achse des Hauptobjektivs
- 2b - 2g: optische Achsen
- 4: Beleuchtungseinrichtung
- 4a: Beleuchtungsachse
- 4b: Haupterstreckungsrichtung der Beleuchtungseinrichtung
- 4c: Umlenkelement der Beleuchtungseinrichtung
- 5, 6, 7, 7a, 8: Umlenkelemente
- 9: optischer Teiler
- 10: Assistentenmikroskop
- 11: optische Komponenten bzw. Zwischenabbildungssysteme
- 12: Vergrößerungssystem (für Hauptoperateur)
- 13: Vergrößerungssystem für Assistenten
- 14a: Umlenkelement
- 15: optische Komponenten
- 16: optische Komponenten für Assistent
- 17a - 17g: Beobachtungsstrahlengänge
- 18: Umlenkelement
- 19: gestrichelte Linie
- 20: Umlenkelement
- 21: Drehpfeil
- 31, 32, 33, 34, 36, 37, 38: Pfeile
- 100: Mikroskop

## Patentansprüche

1. Mikroskop, insbesondere Stereomikroskop, zur Simultanbeobachtung eines Objektes durch einen ersten und einen zweiten Beobachter, mit einem eine optische Achse (2a) aufweisenden Hauptobjektiv (2) und Umlenkelementen (5, 6, 7) zum Umlenken eines parallel zu der optischen Achse (2a) des Hauptobjektivs (2) verlaufenden Beobachtungsstrahlenganges in eine erste Ebene I, welche sich unter einem Winkel, insbesondere im wesentlichen senkrecht, zu der optischen Achse (2a) des Objektivs (2) erstreckt und anschließend in eine zweite Ebene II, welche sich im wesentlichen parallel zu der ersten Ebene I oberhalb von dieser erstreckt, und einer Auskopplungseinrichtung (7) zur Auskopplung eines Beobachtungsstrahlenganges des zweiten Beobachters von dem Beobachtungsstrahlengang des ersten Beobachters,
**gekennzeichnet durch**
eine Umlenkeinrichtung (20) zur Umlenkung des Beobachtungsstrahlenganges des zweiten Beobachters in eine dritte Ebene III, welche sich im wesentlichen parallel zu der ersten und der zweiten Ebene I, II erstreckt, wobei die zweite Ebene II zwischen der ersten Ebene I und der dritten Ebene III liegt.

2. Mikroskop nach Anspruch 1, **dadurch gekennzeichnet, daß** ein Vergrößerungssystem (12) für den ersten Beobachter in der zweiten Ebene II, und ein Vergrößerungssystem (13) für den zweiten Beobachter in der dritten Ebene III angeordnet ist.

3. Mikroskop nach Anspruch 2, **dadurch gekennzeichnet, daß** wenigstens eines der Vergrößerungssysteme (12, 13) eine Zoom-Optik aufweist.

4. Mikroskop nach einem der vorstehenden Ansprüche, **dadurch gekennzeichnet, daß** die Beobachtungsstrahlengänge in der zweiten Ebene II und/oder der dritten Ebene III mittels Drehung der Umlenkelemente (7) bzw. (20) drehbar ausgebildet sind.

5. Mikroskop nach einem der vorstehenden Ansprüche, **dadurch gekennzeichnet, daß** wenigstens eine der Umlenkeinrichtungen (6, 7, 20) halbdurchlässig ausgebildet ist.

6. Mikroskop nach einem der vorstehenden Ansprüche, **dadurch gekennzeichnet, daß** eine Aufspaltung eines das Hauptobjektiv durchstrahlenden Beobachtungsstrahlenganges in wenigstens zwei stereoskopische Beobachtungsstrahlengänge in dem Vergrößerungssystem (12) und/oder dem Vergrößerungssystem (13) erfolgt.

7. Mikroskop nach einem der vorstehenden Ansprüche, **gekennzeichnet durch** eine elektrische und/oder mechanische Kopplung der Vergrößerungssysteme (12, 13).
